# EUROPEAN PATENT APPLICATION

(11) **EP 3 545 946 A1**
(43) Date of publication of application: **02.10.2019**
(21) Application number: 17874147.6
(22) Date of filing: 27.11.2017
(51) Int. Cl.: A61K 8/9789, A61Q 19/00, A61Q 19/08, A61K 36/76

(54) **ANTI-CELLULAR SENESCENCE COSMETIC COMPOSITION, USE OF CASEARIA SYLVESTRIS AND/OR HYMENAEA COURBARIL DERIVATIVES, METHOD FOR PREVENTING CELLULAR SENESCENCE AND METHOD FOR MODULATING THE EXPRESSION OF B-GALACTOSIDASE, P16, P21 AND/OR IL-8**

(30) Priority: 28.11.2016 US 201615362256
(71) Applicant: Natura Cosméticos S.A., 05106-000 São Paulo SP (BR)
(72) Inventor: ARROTEIA, Kelen Fabiola, 05106-000 São Paulo - SP (BR); BUFALO, Michele Cristiane, 18520-000 Cerquilho - SP (BR); PEDROSO DE OLIVEIRA, Ana Paula, 05106-000 São Paulo - SP (BR); ZIMBARDI, Daniela, 05106-000 São Paulo - SP (BR)
(74) Representative: Simmons & Simmons
(86) International application number: PCT/BR2017/050361
(87) International publication number: WO 2018/094495

(57) **Abstract**

The present invention relates to an anti-cell senescence cosmetic composition that comprises one or more derivatives of *Casearia sylvestries, Hymenaea courbaril,* or mixtures thereof and cosmetically acceptable excipients, as well as uses thereof and related prevention methods.

## Description

### FIELD OF THE INVENTION

The present invention relates to an anti-cell senescence cosmetic composition that comprises one or more derivatives of *Casearia sylvestris, Hymenaea courbaril* or mixtures thereof and cosmetically acceptable excipients, as well as uses and related prevention methods.

### BACKGROUND OF THE INVENTION

Cellular senescence is the physiological mechanism that defines the aging at cellular level. It is an irreversible condition, in which a cell undergoes a decline in its biologic competence.

A senescent cell, in spite of being live, is poor in vitality, becoming poorly functional, having little energy and becoming incapable of protecting itself against aggressions from the environment.

In general, a cell in this condition contributes to the decline of the functions of the skin, and the accumulation thereof is related to the various aging signs.

Environmental conditions, such as exposure to sunlight may anticipate the early appearance of these cells in the skin.

Therefore, preventing the occurrence of the senescence process, protecting the cells from the factors that trigger this phenomenon, is a way to delay the appearance of the molecular and visible signs of aging, keeping the cells viable and competent for a longer time. Thus, there is the need for technological alternatives to prevent and, as a result, delay the cell senescence process.

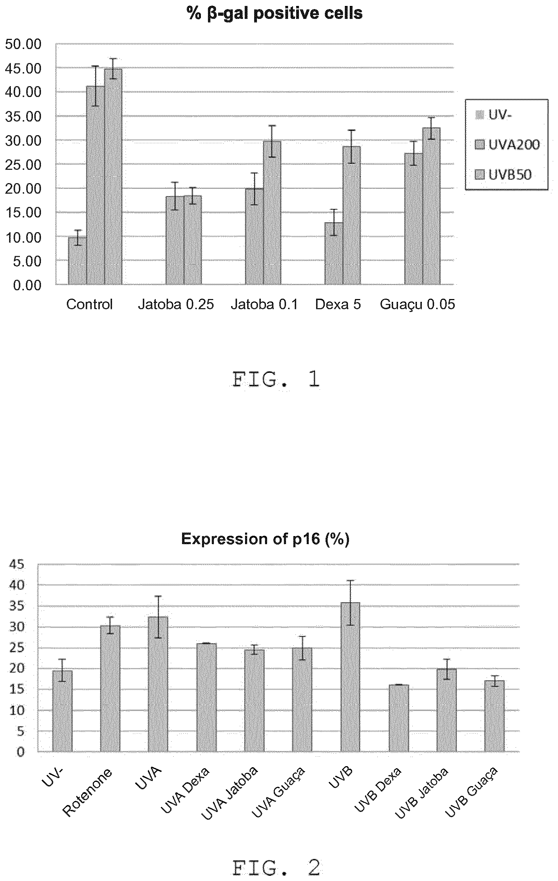

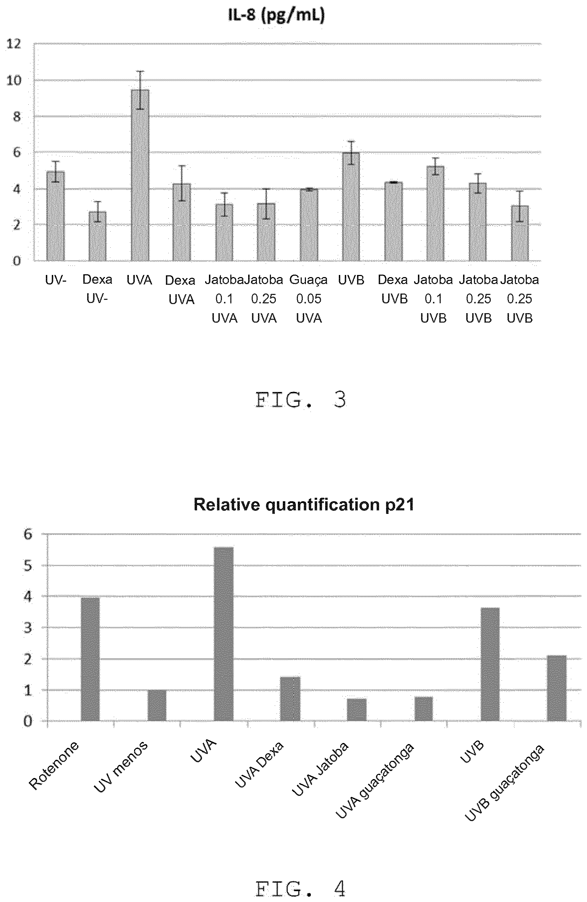

Figures 1 to 4 show comparatively the effects of the active principles according to the present invention at different percentages on the expression of b-galactosidase, p16, IL-8 and p21, respectively. In the graphs, mention of "dexa" means dexamethasone, as well as "guaça" or "guaçatonga" means *Casearia sylvestris* and "jatoba" means *Hymenaea courbaril.*

### DESCRIPTION OF THE INVENTION

Surprisingly, the applicant found that two specific cosmetic ingredients, either alone or in combination, are efficient in preventing cellular senescence by acting directly on the modulation of the expression of b-galactosidase, p16, p21 and IL-8, exhibiting anti-aging (anti-cell senescence), anti-inflammatory effect, as well as antioxidant, stimulating hyaluronic and prebiotic acids, acting on the production of collagen and elastin and as an anti-glycation agent.

Thus, the present invention deals with an anti-cell senescence cosmetic composition comprising one or more derivatives of *Casearia sylvestris, Hymenaea courbaril,* or mixtures thereof and cosmetically acceptable excipients.

*Casearia sylvestris* is popularly known as guagatonga and *Hymenaea courbaril* is popularly known as "jatobá". By "derivatives" in the present invention one means oils, extracts or any fractions derived from them.

The composition according to the present invention particularly comprises about 0.01 to about 0.2 mg/ml of *Casearia sylvestris* derivatives and/or about 0.1 to about 10. Mg/ml of *Hymenaea courbaril* derivatives.

The cosmetically acceptable excipients are those known from the prior art, for instance, included in *The International Cosmetic Ingredient Dictionary and Handbook* (INCI).

In a second embodiment the present invention contemplates the use of derivatives of *Casearia sylvestrie* and/or *Humenaea courbaril* as anti-cell senescence ingredients, particularly in preparing a cosmetic composition for said purpose.

Said ingredients act on the modulation of the expression of b-galactosidase, p16, p21 and/or IL-8, providing active principles that are efficient as anti-aging and anti-inflammatory agents.

In another embodiment, the present invention also contemplates a method of preventing cell senescence, as well as a method for modulating the expression of b-galactosidase, P16, P21 and/or IL-8, which comprises applying onto the skin an effective amount of a cosmetic composition comprising one or more derivatives of *Casearia sylvestris, Humenaea courbaril,* or mixtures thereof and cosmetically acceptable excipients, particularly in the amounts of about 0.01 to about 0.2 mg/ml of derivatives of *Casearia sylvestris* and about 0.1 to about 1.0 mg/ml of derivatives of *Hymenaea courbaril.*

The examples given hereinafter, without imposing any limitation, illustrate the object of the present invention, as well as its effects described herein.

### EXAMPLES

An *in vitro* model of induced early cell aging (cell senescence) was developed and used to evaluate whether certain cosmetic ingredients can protect or delay molecular mechanisms that lead to aging of skin cells or to loss of functional competence (loss of cellular vitality).

The *in vitro* model developed for cellular aging/senescence does not lead to cell death by apoptosis. Besides, protecting the cells from the induced damage of UVA/UVB does not divert the cell from senescence to an event of cell death (reduces senescence without leading to the death pathway).

The *in vitro* model developed for cell aging/senescence does not lead to the expression of MYC (tumorigenic marker, that is, it does not induce oncogenesis). The protection of the cells against induced damage by UVA/UVB does not divert the cell from senescence to an event of oncogenicity/tumorigenesis.

The *in vitro* model developed for cell aging/senescence induces increase of cell senescence-associated lysosomal b-galactosidase (flow cytometry and cytochemical marking) and the appearance of marking induced by UV is reduced in the presence of agents: Dexamethasone (anti-inflammatory), trolox (anti-oxidant) and caffeic acid.

The cell size was also evaluated, since b-gal positive and p16 positive cells exhibit an increase in size and volume.

It was also found that the exposure of the cells to the senescence model raises the expression of the transcription factor p16 (interruption of the cell cycle). The protection of the cells with caffeic acid, dexamethasone and trolox reduces the induced expression of p16.

The exposure of the cells to the senescence model raises the expression of the transcription factor p21. The protection of the cells with dexamethasone and trolox reduces the induced expression of p21.

The inflammatory cytokine IL-8 is modulated in the senescence model. The expression increases with UVA and is reduced in the presence of UVA + trolox, or dexamethasone or caffeic acid.

The reduction of P21, P16, b-gal, cell size, IL-8 was confirmed in the tests carried out with *Casearia sylvestris* and *Hymenaea courbaril* at concentrations of 0.01 to 0.2 mg/ml and 0.1 to 1mg/ml, respectively.

Figures 1 to 4 show comparatively the effects of the active principles according to the present invention at different percentages in the expression of b-galactosidase, p16, IL-8, and p12, respectively. In the graphs, "dexa" means dexamethasone, as well as "guaça" or "guaçatonga" means *Casearia sylvestris* and "jatobá" means *Hymenaea courbaril.*

A person skilled in the art will promptly know how to evaluate, by means of the teachings contained in the text and in the examples prese3nted, advantages of the invention, and propose variations and equivalent alternatives to embodiments, without departing from the scope of the invention as defined in the accompanying claims

## Claims

1. An anti-cell senescence cosmetic composition, **characterized by** comprising one or more derivatives of *Casearia sylvestris, Hymenaea courbaril,* or mixtures thereof and cosmetically acceptable excipients.

2. The composition according to claim 1, **characterized by** comprising about 0.01 to about 0.2 mg/ml of derivatives of *Casearia sylvestris.*

3. The composition according to claim 1, **characterized by** comprising about 0.1 to about 1.0 mg/ml of derivatives of *Hymenaea courbaril.*

4. Use of derivatives of *Casearia sylvestris* and/or *Humenaea courbaril,* **characterized by** being in the preparation of an anti-cell senescence cosmetic composition.

5. Use of derivatives of *Casearia sylvestris* and/or *Humenaea courbaril,* **characterized by** being in anti-cell senescence.

6. The use according to any one of claims 4 and 5, **characterized by** being in the expression of b-galactosidase, p16, p21 and/or IL-8.

7. The use according to any one of claims 4 or 5, **characterized by** being anti-aging and anti-inflammatory.

8. A method of preventing cell senescence, **characterized by** comprising applying to the skin an effective amount of a cosmetic composition comprising one of more derivatives of *Casearia sylvestris, Hymenaea courbaril,* or mixtures thereof and cosmetically acceptable excipients.

9. The method according to claim 7, **characterized in that** the composition comprises about 0.01 to about 0.2 mg/ml of derivatives of *Casearia sylvestris.*

10. The method according to claim 7, **characterized in that** the composition comprises about 0.1 to about 1.0 mg/ml of derivatives of *Hymenaea courbaril.*

11. A method for modulationg the expression of b-galactosidase, p16, p21 and/or IL-8, **characterized by** comprising applying to the skin an effective amount of a cosmetic composition comprising one or more derivatives of *Casearia sylvestries, Humenaea courbaril,* or mixtures thereof and cosmetically acceptable excipients.

12. The method according to any one of claims 8 or 11, **characterized by** being anti-aging and anti-inflammatory.
